(19) Europäisches Patentamt
European Patent Office
Office européen des brevets

(11) Veröffentlichungsnummer: **0 023 560**
**A1**

(12) # EUROPÄISCHE PATENTANMELDUNG

(21) Anmeldenummer: **80103416.6**

(22) Anmeldetag: **19.06.80**

(51) Int. Cl.³: **C 07 C 131/00,** C 07 C 131/02

(30) Priorität: **05.07.79 DE 2927117**

(43) Veröffentlichungstag der Anmeldung: **11.02.81**
**Patentblatt 81/6**

(84) Benannte Vertragsstaaten: **AT BE CH DE FR GB IT LI LU NL SE**

(71) Anmelder: **BASF Aktiengesellschaft,**
**Carl-Bosch-Strasse 38, D-6700 Ludwigshafen (DE)**

(72) Erfinder: **Linhart, Friedrich, Dr., Leisberg 61,**
**D-6900 Heidelberg (DE)**
Erfinder: **Girgensohn, Bjoern, Dr., Neckarpromenade 38,**
**D-6800 Mannheim (DE)**

(54) **Verfahren zur Herstellung von O-substituierten Ketoximen.**

(57) Herstellung von O-substituierten Ketoximen durch Umsetzung der Alkalisalze von Ketoximen mit Alkylierungsmitteln in Gegenwart aprotisch-dipolarer Lösungsmittel.

Die nach dem Verfahren der Erfindung herstellbaren O-substituierten Ketoxime sind wertvolle Ausgangsstoffe für die Herstellung von Farbstoffen, Pflanzenschutzmitteln und Pharmazeutika.

EP 0 023 560 A1

# Verfahren zur Herstellung von O-substituierten Ketoximen

Die Erfindung betrifft ein Verfahren zur Herstellung von O-substituierten Ketoximen durch Umsetzung der Alkalisalze von Ketoximen mit Alkylierungsmitteln in Gegenwart aprotisch-dipolarer Lösungsmittel.

Es ist aus Houben-Weyl, Methoden der Organischen Chemie, Band 10/4, Seiten 217 bis 223 bekannt, daß bei der Umsetzung von Ketoximen mit einem Alkylierungsmittel die Alkylgruppe sowohl an den Stickstoff als auch an den Sauerstoff treten kann. Im ersten Fall erhält man ein Nitron, im zweiten Fall einen Oximäther. Meistens findet man beide Reaktionsprodukte nebeneinander. Es werden Methoden beschrieben, mit deren Hilfe man diese Endstoffe ausschließlich erhalten kann, doch sind diese Methoden entweder großtechnisch nicht durchführbar oder liefern den gewünschten Stoff nur in ungenügender Ausbeute. So lehrt Houben-Weyl (loc. cit., Seite 223), daß das getrocknete Silbersalz des jeweiligen Oxims in absolutem Äther oder Alkohol mit Alkyljodid in Gegenwart von Silberoxid umgesetzt werden. Die Arbeitsweise ist umständlich und großtechnisch unwirtschaftlich. Man kann auch Cyclohexanonoxim in wäßriger Natronlauge mit Dimethylsulfat methylieren (Coll. Czech. Chem. Comm. 14, 561 - 563 (1949)). Die Ausbeute beträgt aber nur 37 % der Theorie an noch unreinem Endstoff. Die Verwendung des hochtoxischen Dimethylsulfats schafft außerdem zusätzliche sicherheitstechnische Probleme. Auch im Falle der Umsetzung mit Allylbromid erhält man lediglich eine Ausbeute von 35,9 % der Theorie.

WB/BL

BAD ORIGINAL

Es wurde nun gefunden, daß man O-substituierte Ketoxime der Formel

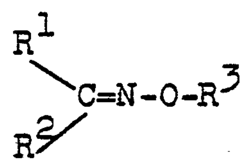 I,

worin die einzelnen Reste $R^1$, $R^2$ und $R^3$ gleich oder verschieden sein können und jeweils einen aliphatischen, cycloaliphatischen oder araliphatischen Rest bedeuten, $R^1$ und $R^2$ auch jeweils einen aromatischen Rest bezeichnen oder zusammen mit dem benachbarten Kohlenstoffatom für Glieder eines alicyclischen Ringes stehen können, durch Umsetzung von Ketoximen mit Alkylierungsmitteln in Gegenwart von Lösungsmitteln vorteilhaft erhält, wenn man Alkalisalze von Ketoximen der Formel

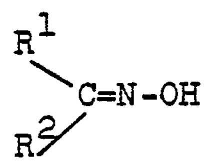 II,

worin $R^1$ und $R^2$ die vorgenannte Bedeutung besitzen, mit Alkylierungsmitteln der Formel

$$R^3\text{-}X \qquad \text{III},$$

worin $R^3$ die vorgenannte Bedeutung besitzt und X ein Chloratom oder ein Bromatom bedeutet, in Gegenwart von aprotisch-dipolaren Lösungsmitteln umsetzt.

Weiterhin wurde gefunden, daß man vorteilhaft mit den Ketoximen der Formel II und Alkalihydroxid anstelle der Alkalisalze der Ketoxime II umsetzt.

BAD ORIGINAL

Die Umsetzung kann für den Fall der Verwendung des Alkalisalzes von Cyclohexanonoxim und Methylchlorid durch die folgenden Formeln wiedergegeben werden:

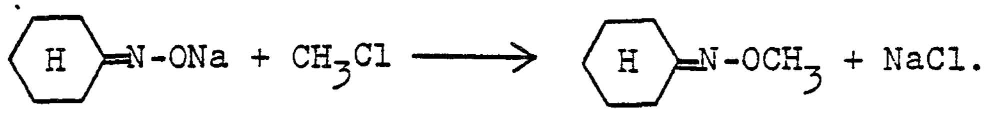

Im Vergleich zu den bekannten Verfahren liefert das Verfahren nach der Erfindung auf einfacherem und wirtschaftlicherem Wege O-substituierte Ketoxime in besserer Ausbeute und Reinheit. Da auch weniger Nebenprodukte anfallen, ist die Entsorgung umweltfreundlicher und einfacher. Die Verwendung toxischer Verbindungen wie Dimethylsulfat wird vermieden. Alle diese vorteilhaften Ergebnisse sind im Hinblick auf den Stand der Technik überraschend.

Bevorzugte Ausgangsstoffe II und III und dementsprechend bevorzugte Endstoffe I sind solche, in deren Formeln die einzelnen Reste $R^1$, $R^2$ und $R^3$ gleich oder verschieden sein können und jeweils einen Alkylrest mit 1 bis 7 Kohlenstoffatomen, einen Alkenylrest mit 3 bis 7 Kohlenstoffatomen, einen Cycloalkylrest mit 5 bis 8 Kohlenstoffatomen, einen Aralkylrest mit 7 bis 12 Kohlenstoffatomen bedeuten, $R^1$ und $R^2$ auch jeweils einen Alkylarylrest mit 7 bis 12 Kohlenstoffatomen oder einen Phenylrest oder zusammen mit dem benachbarten Kohlenstoffatom für Glieder eines 5- bis 8-gliedrigen alicyclischen Ringes stehen können und X ein Bromatom oder Chloratom bedeutet. Die vorgenannten Reste und Ringe können noch durch unter den Reaktionsbedingungen inerte Gruppen, z.B. Alkylgruppen mit 1 bis 4 Kohlenstoffatomen, substituiert sein. Als Alkalisalze der Ausgangsstoffe II sind Natrium- und Kaliumsalze vorteilhaft.

BAD ORIGINAL

Als Ausgangsstoffe kommen z.B. die Alkalisalze folgender Ketoxime II in Betracht: Das Natriumsalz von Acetonoxim, Diäthyl-, Dipropyl-, Diisopropyl-, Dibutyl-, Diisobutyl-, Di-sek.-butyl-, Di-tert.-butyl-, Dicyclopentyl-, Dicyclohexyl-, Dibenzyl-, Diphenyl-, Ditolyl-, Methyläthyl-, Diallyl-, Benzylmethyl-ketonoxim, Cyclohexanon-, Cyclopentanon-, Cycloheptanon-, Cyclooctanon-oxim; analoge Kaliumsalze.

Als Ausgangsstoffe III sind z.B. geeignet: Methyl-, Äthyl-, Propyl-, Isopropyl-, Butyl-, Isobutyl-, sek.-Butyl-, tert.-Butyl-, Allyl-, Cyclohexyl-, Cyclopentyl-, Cycloheptyl-, Benzyl-chlorid; entsprechende Bromide. Alkylhalogenide und Alkenylhalogenide III sind bevorzugt.

Die Umsetzung wird im allgemeinen bei einer Temperatur von -20 bis +100°C, vorzugsweise von 0 bis 80°C, insbesondere bei 20 bis 50°C, drucklos oder unter Druck, kontinuierlich oder diskontinuierlich durchgeführt. Man setzt die Ausgangsstoffe in stöchiometrischer Menge oder im Überschuß, zweckmäßig in einer Menge von 1 bis 2, insbesondere von 1,05 bis 1,5 Mol Ausgangsstoff III je Mol Ausgangsstoff II, um. Als aprotisch-dipolare Lösungsmittel werden hier solche Lösungsmittel definiert, bei denen ein Lösungsmittelmolekül ein ausgeprägtes Dipolmoment besitzt, jedoch keine Wasserstoffatome trägt, welche zur Ausbildung von Wasserstoffbrücken befähigt sind. Die Dielektrizitätskonstante solcher Lösungsmittel ist größer als 15. Bezüglich der Definition für aprotisch-dipolare Lösungsmittel wird auf A. J. Parker, Chem. Rev. 69 (1969), Seiten 1 - 32, insbesondere Seite 2, verwiesen. So sind beispielsweise geeignete aprotisch-dipolare Lösungsmittel Sulfoxide wie

Dimethylsulfoxid, Diäthylsulfoxid, Dimethylsulfon, Diäthylsulfon, Methyläthylsulfon, Tetramethylensulfon; Nitrile wie Acetonitril, Benzonitril, Butyronitril, Isobutyronitril, m-Chlorbenzonitril; N,N-disubstituierte Carbonamide wie Dimethylformamid, Tetramethylharnstoff, N,N-Dimethylbenzamid, N,N-Dimethylacetamid, N,N-Dimethylphenylacetamid, N,N-Dimethylcyclohexancarbonsäureamid, N,N-Dimethylpropionsäureamid und homologes Carbonsäurepiperidid, Carbonsäuremorpholid, Carbonsäurepyrrolidid; entsprechende N,N-Diäthyl-, N,N-Dipropyl-, N,N-Diisopropyl-, N,N-Diisobutyl-, N,N-Dibenzyl-, N,N-Diphenyl-, N-Methyl-N-phenyl-, N-Cyclohexyl-N-methyl-, N-Äthyl-N-tert.-butyl-verbindungen, N-Methyl-formanilid, N-Äthylpyrrolidon, N-Butylpyrrolidon, N-Äthyl-piperidon-(6), N-Methylpyrrolidon; Hexamethylphosphorsäuretriamid; und entsprechende Gemische. Bevorzugt sind Dimethylacetamid, N-Methylpyrrolidon, Hexamethylphosphorsäuretriamid, Dimethylsulfoxid und Tetramethylensulfon. Zweckmäßig verwendet man das Lösungsmittel in einer Menge von 100 bis 10 000 Gewichtsprozent, vorzugsweise von 400 bis 2 000 Gewichtsprozent, bezogen auf Ausgangsstoff II.

Die Reaktion kann wie folgt durchgeführt werden: Ein Gemisch des Alkalisalzes von Ausgangsstoff II, Alkylierungsmittel III und Lösungsmittel wird während 1 bis 20 Stunden bei der Reaktionstemperatur gehalten. Dann wird aus dem Reaktionsgemisch der Endstoff in ü blicher Weise, z.B. durch Zugabe von Wasser, Extraktion des wäßrigen Gemischs, z.B. mit Cyclohexan, und Destillation der vereinigten organischen Phasen, abgetrennt. Die Umsetzung des Alkalisalzes des Ketoxims II mit dem Alkylierungsmittel III kann man zweckmäßig so durchführen, daß man das Salz, in dem gewählten aprotisch-dipolaren Lösungsmittel gelöst oder suspendiert, vorlegt und das Alkylierungsmittel III in Substanz, gelöst oder als Gas eingibt. Man kann aber

auch so verfahren, daß man das Alkylierungsmittel III, gegebenenfalls im Lösungsmittel gelöst, vorlegt und mit dem Alkalisalz des Ketoxims versetzt. Zur Abtrennung des Endstoffs kann man auch das ausgefallene Alkalihalogenid abfiltrieren und den Endstoff destillativ aus dem Filtrat isolieren.

In einer zweiten, bevorzugten Arbeitsweise des erfindungsgemäßen Verfahrens wird das Alkalisalz des Ketoxims nicht gesondert hergestellt. Auf diese Weise erreicht man eine starke Energie- und Arbeitseinsparung des Verfahrens, die keine oder nur eine geringfügige Verminderung der Ausbeute zur Folge hat. Zweckmäßig setzt man bei dieser Arbeitsweise anstelle des Alkalisalzes des Ketoxims II das Ketoxim II selbst und Alkalihydroxid, vorzugsweise Natriumhydroxid oder Kaliumhydroxid, zu und führt die Umsetzung in vorgenannter Weise durch.

Im allgemeinen vermeidet man, polare, protische Lösungsmittel zuzusetzen. Gegebenenfalls kann ein Anteil an Wasser, das beispielsweise durch das Ketoxim II eingeschleppt wurde, in der Regel nicht mehr als 10, vorteilhaft nicht mehr als 6 Gewichtsprozent, bezogen auf Ausgangsstoff II, anwesend sein. Das Alkalisalz des Ketoxims II kann in bekannter Weise, z.B. durch Umsetzung des Ketoxims II mit alkoholischen Alkalialkoholatlösungen, zweckmäßig 20- bis 40-gewichtsprozentigen, methanolischen Alkalimethylatlösungen, vorteilhaft in Gegenwart unter den Reaktionsbedingungen inerter Lösungsmittel, z.B. aromatischer Kohlenwasserstoffe wie Toluol, hergestellt werden. Die Herstellung des Salzes erfolgt zweckmäßig bei 0 bis 80°C, während 0,5 bis 5 Stunden und mit 50 bis 1 000 Gewichtsprozent inertem Lösungsmittel, bezogen auf Ketoxim II. Das Molverhältnis Alkalialkoholat zu Ketoxim II ist zweckmäßig 1 bis 1,05. Es ist unerheblich, in welchem Lösungs-

mittel und mit welcher Base man das Alkalisalz herstellt, vorausgesetzt, daß die verwendete Base stark genug ist, um aus dem Oxim ein Salz zu bilden. Beim Trocknen des Salzes werden zweckmäßig sämtliche Anteile von protischen Lösungsmitteln, z.B. von Wasser oder Alkoholen, vollständig entfernt. Die Trocknung des Alkalioximates kann bei Raumtemperatur oder erhöhter Temperatur vorgenommen werden, wobei man zur Intensivierung der Trocknung auch verminderten Druck anwenden kann.

Die nach dem Verfahren der Erfindung herstellbaren O-substituierten Ketoxime I sind wertvolle Ausgangsstoffe für die Herstellung von Farbstoffen, Pflanzenschutzmitteln und Pharmazeutika. So kann man durch Hydrierung die O,N-disubstituierten Hydroxylamine herstellen. Solche Hydroxylamine ergeben z.B. nach Umsetzung mit Diketen und α-Hydroxyaldehyden die in der US-Patentschrift 3 993 772 beschriebenen N-Methoxy-N-cycloalkyl-2-methyl-3-furancarboxamide und die in der deutschen Patentschrift 24 55 082 beschriebenen O-Methyl-N-cyclohexyl-2,5-dimethylfuran-3-hydroxamsäure. Diese Stoffe sind, wie in den beiden Patentschriften erläutert wird, aufgrund ihrer Wirkung gegen pflanzenpathogene Pilze wertvolle Fungizide und finden Anwendung als Pflanzenschutzmittel, Saatgutbeizmittel und Holzschutzmittel.

Die in den folgenden Beispielen aufgeführten Teile bedeuten Gewichtsteile.

Beispiel 1

a) Herstellung des Natriumsalzes des Cyclohexanonoxims: Eine Lösung, bestehend aus 113 Teilen wasserfreiem Cyclohexanonoxim und 180 Teilen 30-gewichtsprozentiger, methanolischer Natriummethylatlösung in 800 Teilen

Toluol wird im Vakuum bis zur Trockene eingeengt. Man erhält 138 Teile Natriumsalz des Cyclohexanonoxims vom Fp 140°C (unter Zersetzung).

b) Umsatzung: In eine Suspension aus 138 Teilen des Natriumsalzes des Cyclohexanonoxims in 500 Teilen N-Methylpyrrolidon werden bei 40°C in 2 Stunden 60 Teile Methylchlorid eingeleitet. Nach einstündigem Rühren bei 40°C gibt man 1 000 Teile Wasser zu, trennt die organische Phase ab, extrahiert die Wasserphase einmal mit Cyclohexan und unterwirft die vereinigten organischen Phasen einer fraktionierten Destillation. Man erhält 115,5 Teile (91 % der Theorie) O-Methylcyclohexanonoxim vom Siedepunkt 48 bis 50°C bei 13 mbar.

Beispiel 2

In eine Suspension von 1 075,5 Teilen wasserhaltigem (5,5 Gew.-% Wasser) Cyclohexanonoxim und 468 Teilen pulverförmigem Natriumhydroxid in 3 150 Teilen N-Methylpyrrolidon werden bei 50°C in 2,5 Stunden 591 Teile Methylchlorid eingeleitet. Nach einstündigem Rühren wird das Gemisch analog Beispiel 1b) aufgearbeitet. Man erhält 915 Teile O-Methylcyclohexanonoxim (82 % der Theorie) vom Siedepunkt 48 bis 50°C bei 13 mbar.

Beispiel 3

Zu 357 Teilen 95-gewichtsprozentigem (5 Gew.-% Wasser) Cyclohexanonoxim in 1 100 Teilen Dimethylacetamid gibt man 180 Teile pulverförmiges Natriumhydroxid und leitet bei 50°C unter gutem Rühren 400 Teile Chlormethan durch das Gemisch. Man läßt das Gemisch 12 Stunden bei 25°C rühren und vermischt es dann mit 2 000 Teilen Wasser. Das Gemisch wird mit Cyclohexan extrahiert und der Extrakt fraktioniert destilliert. Man erhält 319 Teile O-Methylcyclohexanonoxim (84 % der Theorie) vom Siedepunkt 48 bis 50°C bei 13 mbar.

Beispiel 4

Zu einer Suspension von 138 Teilen Natriumsalz des Cyclohexanonoxims in 350 Teilen N-Methylpyrrolidon gibt man bei 40°C 130 Teile 2-Brompropan. Anschließend hält man die Reaktionslösung noch 2 Stunden bei dieser Temperatur. Nach Aufarbeitung analog Beispiel 1 erhält man 101,7 Teile O-Isopropylcyclohexanonoxim (66 % der Theorie) vom Kp 77 bis 80°C (27 mbar).

Beispiel 5

Die Herstellung des O-Allylcyclohexanonoxims erfolgt unter Verwendung von 3-Brompropen-(1) statt 2-Brompropan analog Beispiel 4). Man erhält 133 Teile O-Allylcyclohexanonoxim (85 % der Theorie) vom Kp 100 bis 102°C (29 mbar).

Beispiel 6

In eine Suspension von 357 Teilen wasserhaltigem Cyclohexanonoxim (Wassergehalt 5 Gew.-%) und 156 Teilen pulverförmigem Natriumhydroxid in 1 050 Teilen N-Methylpyrrolidon leitet man unter Rühren bei 45°C in 2 Stunden 258 Teile Äthylchlorid. Man läßt das Gemisch noch eine Stunde bei 45°C nachreagieren. Man gibt 2 000 Teile Wasser hinzu und extrahiert die wäßrige Phase mit Cyclohexan. Bei der Destillation ergeben sich 337,5 Teile O-Äthylcyclohexanonoxim (80 % der Theorie) vom Kp 63 bis 65°C (13 mbar).

Beispiel 7

Zu einer Suspension von 357 Teilen wasserhaltigem (5 Gew.%) Cyclohexanonoxim und 156 Teilen pulverförmigem Natriumhydroxid in 1 050 Teilen N-Methylpyrrolidon gibt man unter Rühren bei 40°C 472 Teile Allylbromid. Man rührt das Gemisch eine Stunde bei 40°C nach. Nach Aufarbeitung analog Beispiel 1 erhält man 350,6 Teile (76 % der Theorie) O-Allylcyclohexanonoxim vom Kp 105 bis 107°C (33 mbar).

Beispiel 8

Die Herstellung von O-Benzylcyclohexanonoxim erfolgt analog Beispiel 7 unter Verwendung von Benzylbromid statt Allylbromid. Man erhält 426 Teile O-Benzylcyclohexanonoxim (70 % der Theorie) vom Kp 96 bis 98°C (0,4 mbar).

Beispiel 9

Zu einer Suspension von 95 Teilen Natriumsalz des Acetonoxims (hergestellt durch Einengen einer Lösung aus 73 Teilen Acetonoxim und 180 Teilen 30-gewichtsprozentiger, methanolischer Natriummethylatlösung in 600 Teilen Toluol, im Vakuum) in 350 Teilen N-Methylpyrrolidon gibt man bei 35°C 132,8 Teile Benzylchlorid. Man läßt das Gemisch 30 Minuten bei 35°C nachreagieren. Analog Beispiel 1 erhält man 123 Teile O-Benzylacetonoxim (75 % der Theorie) vom Kp 52 bis 58°C (0,4 mbar).

Beispiel 10

In eine Suspension von 95 Teilen Natriumsalz des Acetonoxims in 350 Teilen N-Methylpyrrolidon leitet man unter Rühren bei 45°C 60,5 Teile Methylchlorid ein. Man läßt das Gemisch 2 Stunden bei 40°C nachreagieren. Nach Auf-

arbeitung analog Beispiel 1) mit n-Pentan anstelle von Cyclohexan, erhält man 53,4 Teile O-Methylacetonoxim (62 % der Theorie) vom Kp 72 bis 73°C

## Patentansprüche

1. Verfahren zur Herstellung von O-substituierten Ketoximen der Formel

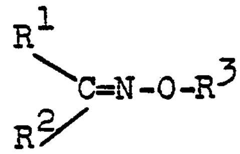

I,

worin die einzelnen Reste $R^1$, $R^2$ und $R^3$ gleich oder verschieden sein können und jeweils einen aliphatischen, cycloaliphatischen oder araliphatischen Rest bedeuten, $R^1$ und $R^2$ auch jeweils einen aromatischen Rest bezeichnen oder zusammen mit dem benachbarten Kohlenstoffatom für Glieder eines alicyclischen Ringes stehen können, durch Umsetzung von Ketoximen mit Alkylierungsmitteln in Gegenwart von Lösungsmitteln, dadurch gekennzeichnet, daß man Alkalisalze von Ketoximen der Formel

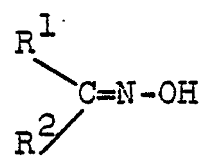

II,

worin $R^1$ und $R^2$ die vorgenannte Bedeutung besitzen, mit Alkylierungsmitteln der Formel

$$R^3\text{-}X \qquad \text{III},$$

worin $R^3$ die vorgenannte Bedeutung besitzt und X ein Chloratom oder ein Bromatom bedeutet, in Gegenwart von aprotisch-dipolaren Lösungsmitteln umsetzt.

2. Verfahren nach Anspruch 1, dadurch gekennzeichnet, daß man mit den Ketoximen der Formel II und Alkalihydroxid anstelle der Alkalisalze der Ketoxime II umsetzt.

Europäisches Patentamt

# EUROPÄISCHER RECHERCHENBERICHT

0023560

Nummer der Anmeldung

EP 80 10 3416.6

| EINSCHLÄGIGE DOKUMENTE | | | KLASSIFIKATION DER ANMELDUNG (Int.Cl.3) |
|---|---|---|---|
| Kategorie | Kennzeichnung des Dokuments mit Angabe, soweit erforderlich, der maßgeblichen Teile | betrifft Anspruch | |
| | DE - B - 1 793 211 (RHONE-POULENC)<br>* Spalte 1, insbesondere Zeilen 35 bis 42 *<br>---- | 1 | C 07 C 131/00<br>C 07 C 131/02 |
| | | | RECHERCHIERTE SACHGEBIETE (Int. Cl.3) |
| | | | C 07 C 131/00 |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt. | | |

| Recherchenort | Abschlußdatum der Recherche | Prüfer |
|---|---|---|
| Berlin | 03-11-1980 | STOOS |

EPA form 1503.1 06.78